(19) Europäisches Patentamt
European Patent Office
Office européen des brevets

(11) EP 0 672 409 B1

(12) **FASCICULE DE BREVET EUROPEEN**

(45) Date de publication et mention de la délivrance du brevet:
**21.05.1997 Bulletin 1997/21**

(51) Int. Cl.$^6$: **A61K 7/48**, A61K 7/06

(21) Numéro de dépôt: **95400185.5**

(22) Date de dépôt: **27.01.1995**

(54) **Compositions cosmétiques contenant un mélange synergétique de polymères conditionneurs**

Kosmetische Zusammensetzungen, die ein synergistisches Gemisch von polymeren Konditionierungsmitteln enthalten

Cosmetic composition containing a synergic mixture of condioning polymers

(84) Etats contractants désignés:
**DE ES FR GB IT**

(30) Priorité: **15.03.1994 FR 9402991**

(43) Date de publication de la demande:
**20.09.1995 Bulletin 1995/38**

(73) Titulaire: **L'OREAL**
**75008 Paris (FR)**

(72) Inventeurs:
- **Cauwet-Martin, Danièle**
  **F-75011 Paris (FR)**
- **Dubief, Claude**
  **F-78150 Le Chesnay (FR)**

(74) Mandataire: **Tezier Herman, Béatrice**
**L'OREAL,**
**Département Propriété Industrielle,**
**90, rue du Gal Roguet**
**92583 Clichy Cédex (FR)**

(56) Documents cités:
**EP-A- 0 308 190**
**EP-A- 0 521 748**
**DE-A- 3 731 477**
**FR-A- 2 680 682**
**GB-A- 2 188 948**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Rank Xerox (UK) Business Services
2.14.3/3.4

## Description

L'invention a pour objet des compositions cosmétiques pour les cheveux et la peau contenant, dans des proportions déterminées convenant à l'obtention d'un effet de synergie, des polymères conditionneurs.

On a déja préconisé dans des compositions pour le lavage ou le soin des cheveux l'utilisation de polymères conditionneurs, notamment cationiques, pour faciliter le démêlage des cheveux et pour leur communiquer douceur et souplesse. Cependant, l'usage des polymères cationiques dans ce but présente divers inconvénients. En raison de leur forte affinité pour les cheveux, certains de ces polymères se déposent de façon importante lors d'utilisations répétées, et conduisent à des effets indésirables tel qu'un toucher désagréable, un raidissement des cheveux, et une adhésion interfibre affectant le coiffage. Ces inconvénients sont accentués dans le cas de cheveux fins, qui manquent de tenue, de nervosité et de volume.

Parmi les documents de l'art antérieur décrivant l'utilisation de polymères cationiques comme agents cosmétiques, on peut citer les brevets US 4 157 388, 4 390 689, 4 702 906 et 4 719 282, qui décrivent l'utilisation de polymères quaternisés.

L'utilisation de ces polymères quaternisés comme seuls agents de traitement cosmétique n'est toutefois pas complètement satisfaisante quant à la tenue des cheveux.

On a également préconisé pour améliorer les propriétés conditionnantes des produits capillaires, l'utilisation de polymères amphotères tels que ceux décrits dans la demande de brevet EP-A-269 243. Cependant, les compositions contenant uniquement ces polymères ne permettent pas d'obtenir une douceur et un démêlage suffisants.

Par ailleurs, dans les demandes FR-A-2 470 596 et FR-A-2 519 863, il est prévu des compositions cosmétiques pour le traitement des cheveux contenant l'association d'un polymère cationique et d'un polymère amphotère. Si ces compositions sont supérieures aux compositions ne contenant qu'un polymère cationique ou qu'un polymère amphotère, elles ne donnent cependant pas entière satisfaction en ce qui concerne les propriétés de démêlage et de douceur conférées aux cheveux.

Or, la demanderesse a maintenant découvert que l'association de certains polymères conditionneurs décrits dans les documents antérieurs qui viennent d'être mentionnés, lorsqu'ils sont convenablement sélectionnés et utilisés dans des proportions respectives variant dans des limites bien déterminées, permet de remédier à ces inconvénients du fait d'un effet de synergie.

Cette association apporte en effet des propriétés cosmétiques nettement améliorées par rapport aux propriétés obtenues avec l'un ou l'autre des constituants utilisé seul, ainsi que par rapport aux associations des deux constituants utilisés dans des rapports sortant du domaine de l'invention.

On a découvert en particulier que les compositions obtenues selon l'invention apportent une amélioration du démêlage (notamment sur cheveux humides) ainsi qu'une amélioration de la douceur des cheveux. En outre, les cheveux ne sont pas alourdis après des applications répétées.

Par ailleurs, les compositions de l'invention appliquées sur la peau notamment sous forme de bain moussant ou de gel douche, apportent une amélioration de la douceur de la peau.

L'invention a donc pour objet des compositions cosmétiques qui sont essentiellement caractérisées par le fait qu'elles comprennent :

- au moins un polymère (a) de polyammonium quaternaire constitué d'une succession de motifs répondant à la formule (I) suivante :

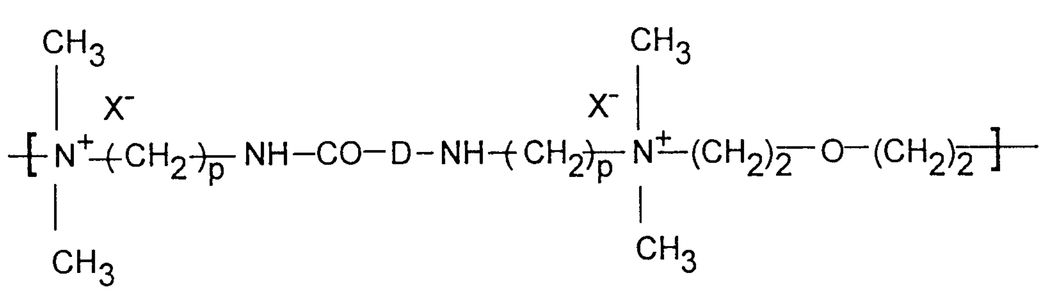

(I)

dans laquelle :

- p désigne un nombre entier variant de 1 à 6 environ,
- D peut être nul ou peut représenter un groupement $-(CH_2)_r-CO-$ dans lequel r désigne un nombre égal à 4 ou à 7,

- $X^-$ est un anion dérivé d'un acide minéral ou organique,

le poids moléculaire dudit polymère (a) étant inférieure à 100 000, de préférence inférieure ou égale à 50 000, et

- au moins un polymère (b) constitué de 70 à 90 % en poids environ d'unités diallyldialkylammonium dans lesquelles le radical alkyle contient de 1 à 18 atomes de carbone et de 30 % à 10 % en poids environ d'unités acrylique ou méthacrylique,

lesdits polymères (a) et (b) étant présents dans une proportion produisant une activité synergique.

Parmi les polymères (a) ci-dessus, on préfère mettre en oeuvre :

i) les polymères pour lesquels D représente un groupement $-(CH_2)_4-CO-$ et X désigne un atome de chlore ; un polymère de ce type est vendu par la société MIRANOL sous le nom de MIRAPOL-AD1®,
ii) les polymères pour lesquels D représente un groupement $-(CH_2)_7-CO-$ et X désigne un atome de chlore ; un polymère de ce type est vendu par la société MIRANOL sous le nom de MIRAPOL-AZ1®,
iii) les polymères pour lesquels D désigne la valeur zéro et X désigne un atome de chlore ; un polymère de ce type est vendu par la société MIRANOL sous le nom de MIRAPOL-A15®,
iiii) les copolymères blocs formés de motifs correspondant aux polymères décrits aux alinéas i) et iii) ci-dessus, et vendus par la société MIRANOL sous les noms de MIRAPOL-9®, MIRAPOL-175® et MIRAPOL-95®.

De tels polymères peuvent être préparés selon les procédés décrits dans les brevets US 4 157 388, 4 390 689, 4 702 906 et 4 719 282.

Parmi les polymères (b), on préfère les copolymères de chlorure de diallyldiméthylammonium ou de diallyldiéthylammonium et d'acide acrylique d'un poids moléculaire compris entre 50 000 et 10 000 000 et de préférence entre 200 000 et 5 000 000.

Un polymère de ce type, et particulièrement préféré selon l'invention, est le copolymère de chlorure de diallyldiméthylammonium et d'acide acrylique (80/20 en poids) vendu en solution à 35 % de matière active par les sociétés CALGON CORP et MERCK sous la dénomination de MERQUAT 280®.

L'effet de synergie entre les polymères (a) et (b) ci-dessus définis apparait généralement dans un rapport pondéral (a)/(b) compris entre 0,1:1 et 3:1 environ. Toutefois, la plage exacte des rapports pondéraux convenables (i.e. pour lesquels l'effet de synergie est effectivement obtenu) peut varier selon la nature du polymère (a) mis en oeuvre. Ainsi, à titre d'exemple, on a pu constater un effet de synergie remarquable sur les propriétés cosmétiques dans les cas particuliers suivants :

- une association entre un polymère (a) tel que défini à l'alinéa iii) ci-dessus et un polymère (b), pour un domaine de rapports pondéraux (a)/(b) compris entre 0,1:1 et 1,5:1,
- une association entre un polymère (a) tel que défini à l'alinéa i) ci-dessus et un polymère (b), ou entre un polymère (a) tel que défini à l'alinéa ii) ci-dessus et un polymère (b), pour un domaine de rapports pondéraux (a)/(b) compris entre 1,25:1 et 1,75:1,
- une association entre un polymère (a) tel que défini à l'alinéa iiii) ci-dessus et un polymère (b), pour un domaine de rapports pondéraux (a)/(b) compris entre 0,5:1 et 3:1.

Dans les compositions selon l'invention, la quantité pondérale du polymère (a) est préférentiellement comprise entre 0,05% et 4%, encore plus préférentiellement entre 0,1% et 3%, et celle du polymère (b) varie de préférence entre 0,1% et 8%, encore plus préférentiellement entre 0,2% et 6%, par rapport au poids total de la composition.

Les compositions de l'invention contiennent en outre avantageusement au moins un agent tensioactif qui est généralement présent en une quantité comprise entre 0,1% et 40% en poids environ, de préférence entre 3% et 40% et encore plus préférentiellement entre 5% et 30%, par rapport au poids total de la composition.

Cet agent tensioactif peut être choisi parmi les agents tensioactifs anioniques, amphotères, zwitterioniques, non-ioniques, cationiques ou leurs mélanges.

Parmi les tensioactifs anioniques, on peut citer les sels (en particulier sels alcalins, notamment de sodium, sels d'ammonium, sels d'amines, sels d'aminoalcools ou sels de magnésium) des composés suivants : les alkyléthersulfates, alkylamidoéthersulfates, alkylarylpolyéthersulfates, monoglycérides sulfates ; les alkylsulfonates, alkylsulfates, alkylphosphates, alkylamidesulfonates, alkylarylsulfonates, $\alpha$-oléfine-sulfonates, paraffine-sulfonates ; les alkylsulfosuccinates, les alkyléthersulfosuccinates, les alkylamide sulfosuccinates ; les alkylsulfosuccinamates ; les alkylsulfoacétates ; les alkylétherphosphates les acylsarcosinates et les N-acyltaurates. Le radical alkyle ou acyle de ces différents composés comporte de préférence de 12 à 20 atomes de carbone.

Parmi les tensioactifs anioniques, on peut encore citer les sels d'acides gras tels que les sels des acides oléique,

ricinoléique, palmitique, stéarique, les acides d'huile de coprah ou d'huile de coprah hydrogénée les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides éthers carboxyliques polyoxyalkylénés, en particulier ceux comportant de 2 à 24 groupements oxyde d'éthylène, et leurs mélanges. Les tensioactifs anioniques du type acide éther carboxylique polyoxyalkyléné sont en particulier ceux qui répondent à la formule (II) suivante :

$$R_1\text{-}(OC_3H_6)_x\text{-}(OC_2H_4)_y\text{-}OCH_2COOA \qquad (II)$$

dans laquelle :

- $R_1$ désigne un groupement alkyle ou alkényle linéaire ou ramifié en $C_8$-$C_{22}$, un groupement alkyl ($C_8$-$C_9$)-phényle, un groupement R'-CONH-$CH_2$- dans lequel R' désigne un radical alkyle ou alkényle en $C_{11}$-$C_{21}$,
- x est un nombre entier ou décimal pouvant varier de 0 à 6, et
- y est un nombre entier ou décimal pouvant varier de 2 à 24 et de préférence de 3 à 10,
- A désigne H, ammonium, Na, K, Li, Mg ou un reste monoéthanolamine ou triéthanolamine. On peut également utiliser des mélanges de composés de formule (II) en particulier des mélanges dans lesquels les groupements $R_1$ sont différents.

Des composés de formule (II) sont vendus par exemple par la Société CHEM Y sous les dénominations AKYPOS® (NP40, NP70, OP40, OP80, RLM25, RLM38, RLMQ 38 NV, RLM 45, RLM 45 NV, RLM 100, RLM 100 NV, RO 20, RO 90, RCS 60, RS 60, RS 100, RO 50) ou par la Société SANDOZ sous les dénominations SANDOPAN® (DTC Acid, DTC).

Selon un mode de réalisation préféré de l'invention, on utilise comme agent tensioactif anionique au moins un composé du type acide carboxylique de formule (II) indiquée ci-dessus, dans laquelle $R_1$ désigne un radical alkyle ($C_{12}$-$C_{14}$), oléyle, cétyle ou stéaryle, A désigne un atome d'hydrogène ou de sodium, x = 0 et y est compris entre 3 et 10. On utilise par exemple le produit commercial vendu par la Société CHEM Y sous la dénomination RLM 45® ($R_1$ = alkyle ($C_{12}$-$C_{14}$) ; valeur moyenne de y = 4,5 ; x = 0 et A = H ).

Les agents tensioactifs non-ioniques peuvent être choisis parmi les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène : les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl ($C_{10}$ - $C_{14}$) amines ou les oxydes de N-acylaminopropylmorpholine. Les alkylpolyglycosides et les polyglycérolés font partie des tensioactifs non-ioniques plus particulièrement préférés.

Les agents tensioactifs amphotères ou zwitterioniques sont notamment des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl ($C_8$-$C_{20}$) bétaïnes, les sulfobétaïnes, les alkyl ($C_8$-$C_{20}$) amidoalkyl ($C_1$-$C_6$) betaïnes ou les alkyl ($C_8$-$C_{20}$) amidoalkyl ($C_1$-$C_6$) sulfobétaïnes.

Parmi les dérivés d'amines, on peut citer les produits vendus sous la dénomination MIRANOL, tels que décrits dans les brevets américains US 2 528 378 et US 2 781 354 et référencés dans le dictionnaire CTFA, 3ème édition, 1982, sous les dénominations de Amphocarboxyglycinates et de Amphocarboxypropionates, de formules respectives :

$$R2\text{-}CONHCH_2CH_2\text{-}N(R3)(R4)(CH_2COO^-)$$

dans laquelle : R2 désigne un radical alkyle d'un acide R2-COOH présent dans l'huile de coprah hydrolysée, un radical heptyle, nonyle ou undécyle, R3 désigne un groupement bêta-hydroxyéthyle et R4 un groupement carboxyméthyle ; et

$$R5\text{-}CONHCH_2CH_2\text{-}N(B)(C)$$

dans laquelle :

B représente $-CH_2CH_2OX'$, C représente $-(CH_2)_z-Y'$, avec z = 1 ou 2,

X' désigne le groupement $-CH_2CH_2-COOH$ ou un atome d'hydrogène,

Y' désigne -COOH ou le radical $-CH_2-CHOH-SO_3H$,

R5 désigne un radical alkyle d'un acide R5-COOH présent dans l'huile de coprah ou dans l'huile de lin hydrolysée, un radical alkyle, notamment en $C_7$, $C_9$, $C_{11}$ ou $C_{13}$, un radical alkyle en $C_{17}$ et sa forme iso, un radical $C_{17}$ insaturé.

A titre d'exemple on peut citer le cocoamphocarboxyglycinate vendu sous la dénomination commerciale MIRANOL C2M® concentré par la Société MIRANOL.

Parmi les tensioactifs cationiques on peut citer en particulier : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkylammonium ou d'alkylpyridinium; les dérivés d'imidazoline ; ou les oxydes d'amines à caractère cationique.

La concentration de ces agents tensio-actifs cationiques est de préférence comprise entre 0,1% et 10% en poids par rapport au poids total de la composition.

Les tensioactifs anioniques sont de préférence utilisés en mélange avec des tensioactifs amphotères. Dans ce cas, le rapport pondéral des premiers aux seconds peut varier de 0,5 à 10 et de préférence de 1 à 5.

Les compositions conformes à l'invention peuvent contenir en outre des adjuvants usuels.

Ce sont par exemple des parfums, des solvants, des agents conservateurs, séquestrants, épaississants, adoucissants, modificateurs de mousse, acidifiants ou alcalinisants.

Les épaississants peuvent être choisis notamment parmi l'alginate de sodium, la gomme arabique, les dérivés cellulosiques tels que la méthylcellulose, l'hydroxyméthylcellulose, l'hydroxyéthylcellulose, l'hydroxypropylcellulose, l'hydroxypropylméthylcellulose, la gomme de guar ou ses dérivés, les gommes de xanthane, les scléroglucanes , les acides polyacryliques réticulés, l'oléate de propylèneglycol oxyéthyléné à 55 moles d'oxyde d'éthylène et des éthers d'alcools gras ayant de 27 à 44 atomes de carbone.

L'épaississant peut également être obtenu par mélange du polyéthylèneglycol et de stéarates ou de distéarates de polyéthylèneglycol ou par mélange d'esters phosphoriques et d'amides.

Ces épaississants sont utilisés de préférence dans des proportions pouvant aller de 0,5 à 5% en poids par rapport au poids total de la composition.

Le milieu aqueux peut contenir, outre de l'eau, des solvants cosmétiquement acceptables tels que des monoalcools, des polyalcools, des éthers de glycol ou des esters d'acides gras, utilisés seuls ou en mélange. Parmi ces solvants, on peut plus particulièrement mentionner les alcools inférieurs tels que l'éthanol, l'isopropanol, les polyalcools tels que l'éthylèneglycol, le diéthylèneglycol, le propylèneglycol, les éthers de glycol et les alkyl éthers de glycol ou de diéthylèneglycol.

Les solvants sont utilisés de préférence dans des proportions comprises entre 0,5 et 10% en poids par rapport au poids total de la composition.

Les compositions selon l'invention peuvent également contenir des colorants, des agents modificateurs de viscosité, des agents nacrants, des agents hydratants, anti-pelliculaires, anti-séborrhéiques, des filtres solaires, des silicones volatiles ou non, organomodifiées ou non, d'autres agents de conditionnement que ceux de l'invention tels que des composés cationiques polymériques ou non, des huiles hydrocarbonées, des protéines, des vitamines, etc.

Le pH des compositions selon l'invention est généralement compris entre 4 et 8 et de préférence entre 5 et 7.

Les compositions conformes à l'invention peuvent être utilisées pour le lavage et le traitement des cheveux et/ou de la peau.

Les compositions de l'invention peuvent plus particulièrement se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Les compositions de l'invention peuvent encore se présenter sous la forme de compositions lavantes pour le corps, et en particulier sous la forme de solutions ou de gels pour le bain ou la douche ou de produits démaquillants.

Les compositions selon l'invention peuvent également se présenter sous forme de lotions aqueuses ou hydroalcooliques pour le soin de la peau et des cheveux.

L'homme de l'art détermine parmi les divers additifs énumérés ci-avant ceux convenant à l'application recherchée.

Les exemples qui suivent vont illustrer l'invention sans toutefois la limiter.

**EXEMPLE 1**

On a préparé diverses lotions démêlantes contenant :

a) un polymère (a), choisi parmi différents produits vendus sous le nom général de MIRAPOL® par la Société MIRANOL, à savoir :

- MIRAPOL 175®
- MIRAPOL A15®
- MIRAPOL AD1®
- MIRAPOL 9®
- MIRAPOL AZ1®

à raison de x g

b) un copolymère (b) de chlorure de diallyldiméthylammonium et d'acide acrylique, à 35 % de matière active (MA), vendu sous la dénomination de MERQUAT 280® par la Société MERCK,
à raison de y g

c) HCl qs pH 5,5

d) Conservateurs qs

e) Eau qsp 100 g

Ces lotions (numérotées ci-après de 1 à 17) présentant des rapports pondéraux x/y entre les polymères (a) et (b) différents ; à chacune de ces lotions, on a fait en outre correspondre des lotions comparatives qui : soit ne contenaient que le polymère (a) à la concentration x + y (lotions de type A), soit ne contenaient que le polymère (b) également à la concentration x + y (lotions de type B).

Les compositions exactes de toutes ces lotions sont données dans le tableau I ci-dessous (les valeurs sont exprimées en g) :

Tableau I

| Lotions / Polymères | 1 | 1A | 1B | 2 | 2A | 2B | 3 | 3A | 3B |
|---|---|---|---|---|---|---|---|---|---|
| (a) : MIRAPOL 175® | 0,1 | 1,1 | | 0,5 | 1,5 | | 3 | 4 | |
| (b) : MERQUAT 280® | 1 | | 1,1 | 1 | | 1,5 | 1 | | 4 |
| Rapport pondéral (a)/(b) | 0,1 | | | 0,5 | | | 3 | | |

Tableau I (suite)

| Lotions / Polymères | 4 | 4A | 4B | 5 | 5A | 5B | 6 | 6A | 6B |
|---|---|---|---|---|---|---|---|---|---|
| (a) : MIRAPOL A15® | 0,1 | 1,1 | | 1,5 | 2,5 | | 2 | 3 | |
| (b) : MERQUAT 280® | 1 | | 1,1 | 1 | | 2,5 | 1 | | 3 |
| Rapport pondéral (a)/(b) | 0,1 | | | 1,5 | | | 2 | | |

Tableau I (suite)

| Lotions / Polymères | 7 | 7A | 7B | 8 | 8A | 8B | 9 | 9A | 9B | 10 | 10 A | 10 B |
|---|---|---|---|---|---|---|---|---|---|---|---|---|
| (a) : MIRAPOL AD1® | 1 | 2 | | 1,25 | 2,25 | | 1,75 | 2,75 | | 2 | 3 | |
| (b) : MERQUAT 280® | 1 | | 2 | 1 | | 2,25 | 1 | | 2,75 | 1 | | 3 |
| Rapport pondéral (a)/(b) | 1 | | | 1,25 | | | 1,75 | | | 2 | | |

Tableau I (suite)

| Lotions / Polymères | 11 | 11A | 11B | 12 | 12A | 12B | 13 | 13A | 13B |
|---|---|---|---|---|---|---|---|---|---|
| (a) : MIRAPOL 9® | 0,5 | 1,5 | | 2 | 3 | | 3 | 4 | |
| (b) : MERQUAT 280® | 1 | | 1,5 | 1 | | 3 | 1 | | 4 |
| Rapport pondéral (a)/(b) | 0,5 | | | 2 | | | 3 | | |

Tableau I (suite et fin)

| Lotions / Polymères | 14 | 14 A | 14 B | 15 | 15 A | 15 B | 16 | 16 A | 16 B | 17 | 17 A | 17 B |
|---|---|---|---|---|---|---|---|---|---|---|---|---|
| (a) : MIRAPOL AZ1® | 1 | 2 | | 1,25 | 2,25 | | 1,75 | 2,75 | | 2 | 3 | |
| (b) : MERQUAT 280® | 1 | | 2 | 1 | | 2,25 | 1 | | 2,75 | 1 | | 3 |
| Rapport pondéral (a)/(b) | 1 | | | 1,25 | | | 1,75 | | | 2 | | |

Le mode opératoire était ensuite le suivant : des mèches identiques présentant chacune 2,5 g de cheveux permanentés ont été respectivement traitées (à raison de 1 g de lotion par mèche) avec chacune des lotions du tableau ci-dessus, puis rincées à l'eau après 2 minutes de pause.

On a ensuite comparé à l'aide d'un test d'évaluation sensorielle, le démêlage à l'état mouillé des cheveux traités par ces lotions.

Le test utilisé a pour objet le classement, par un jury constitué de 10 juges, de chaque série de 3 échantillons (par exemple la série 1, 1A, 1B) en fonction croissante ou décroissante de l'efficacité du démêlage (facilité de passage du peigne). Les 3 échantillons relatif à une même série sont présentées simultanément au juge. On lui demande de les classer des plus faciles à démêler aux plus difficiles. L'analyse statistique des résultats est effectuée à l'aide des tables de A. KRAMER (Food Technology 17 - (12), 124 - 125 1963).

Les résultats sont consignés dans le tableau II donné ci-dessous.

Tableau II

| JUGES / LOTIONS | 1 | 2 | 3 | 4 | 5 | 6 | 7 | 8 | 9 | 10 | SOMME DES RANGS |
|---|---|---|---|---|---|---|---|---|---|---|---|
| 1 | 2 | 2 | 2 | 2 | 2 | 2 | 2 | 2 | 2 | 2 | 20 |
| 1A | 3 | 3 | 3 | 3 | 3 | 3 | 3 | 3 | 3 | 3 | 30 |
| 1B | 1 | 1 | 1 | 1 | 1 | 1 | 1 | 1 | 1 | 1 | 10 |
| 2 | 1 | 1 | 1 | 1 | 1 | 1 | 1 | 1 | 1 | 1 | 10 |
| 2A | 2 | 2 | 2 | 2 | 2 | 2 | 2 | 2 | 2 | 2 | 20 |
| 2B | 3 | 3 | 3 | 3 | 3 | 3 | 3 | 3 | 3 | 3 | 30 |
| 3 | 1 | 2 | 1 | 2 | 1 | 1 | 1 | 1 | 1 | 1 | 12 |
| 3A | 2 | 1 | 2 | 1 | 2 | 2 | 2 | 2 | 2 | 2 | 18 |
| 3B | 3 | 3 | 3 | 3 | 3 | 3 | 3 | 3 | 3 | 3 | 30 |
| 4 | 1 | 1 | 1 | 1 | 1 | 1 | 1 | 1 | 1 | 1 | 10 |
| 4A | 2 | 2 | 2 | 2 | 2 | 2 | 2 | 2 | 2 | 2 | 20 |
| 4B | 3 | 3 | 3 | 3 | 3 | 3 | 3 | 3 | 3 | 3 | 30 |
| 5 | 1 | 1 | 1 | 1 | 1 | 1 | 1 | 1 | 1 | 1 | 10 |
| 5A | 2 | 2 | 2 | 2 | 2 | 2 | 2 | 2 | 2 | 2 | 20 |
| 5B | 3 | 3 | 3 | 3 | 3 | 3 | 3 | 3 | 3 | 3 | 30 |
| 6 | 2 | 2 | 2 | 2 | 2 | 2 | 2 | 2 | 2 | 2 | 20 |
| 6A | 3 | 3 | 3 | 3 | 3 | 3 | 3 | 3 | 3 | 3 | 30 |
| 6B | 1 | 1 | 1 | 1 | 1 | 1 | 1 | 1 | 1 | 1 | 10 |

Tableau II (suite et fin)

| | | | | | | | | | | | |
|---|---|---|---|---|---|---|---|---|---|---|---|
| 7 | 2 | 2 | 2 | 2 | 2 | 2 | 2 | 2 | 2 | 2 | 20 |
| 7A | 3 | 3 | 3 | 3 | 3 | 3 | 3 | 3 | 3 | 3 | 30 |
| 7B | 1 | 1 | 1 | 1 | 1 | 1 | 1 | 1 | 1 | 1 | 10 |
| 8 | 1 | 2 | 1 | 2 | 1 | 1 | 1 | 1 | 1 | 1 | 12 |
| 8A | 3 | 3 | 3 | 3 | 3 | 3 | 3 | 3 | 3 | 3 | 30 |
| 8B | 2 | 1 | 2 | 1 | 2 | 2 | 2 | 2 | 2 | 2 | 18 |
| 9 | 1 | 1 | 2 | 2 | 2 | 1 | 1 | 1 | 1 | 1 | 13 |
| 9A | 2 | 2 | 1 | 1 | 1 | 2 | 2 | 2 | 2 | 2 | 17 |
| 9B | 3 | 3 | 3 | 3 | 3 | 3 | 3 | 3 | 3 | 3 | 30 |
| 10 | 2 | 2 | 2 | 2 | 2 | 2 | 2 | 2 | 2 | 2 | 20 |
| 10A | 1 | 1 | 1 | 1 | 1 | 1 | 1 | 1 | 1 | 1 | 10 |
| 10B | 3 | 3 | 3 | 3 | 3 | 3 | 3 | 3 | 3 | 3 | 30 |
| 11 | 1 | 2 | 1 | 1 | 2 | 1 | 1 | 1 | 1 | 1 | 12 |
| 11A | 2 | 1 | 2 | 2 | 1 | 2 | 2 | 2 | 2 | 2 | 18 |
| 11B | 3 | 3 | 3 | 3 | 3 | 3 | 3 | 3 | 3 | 3 | 30 |
| 12 | 1 | 1 | 1 | 1 | 1 | 1 | 1 | 1 | 1 | 1 | 10 |
| 12A | 2 | 2 | 2 | 2 | 2 | 2 | 2 | 2 | 2 | 2 | 20 |
| 12B | 3 | 3 | 3 | 3 | 3 | 3 | 3 | 3 | 3 | 3 | 30 |
| 13 | 1 | 1 | 1 | 1 | 2 | 1 | 2 | 1 | 1 | 1 | 12 |
| 13A | 3 | 3 | 3 | 3 | 3 | 3 | 3 | 3 | 3 | 3 | 30 |
| 13B | 2 | 2 | 2 | 2 | 1 | 2 | 1 | 2 | 2 | 2 | 18 |
| 14 | 2 | 2 | 2 | 2 | 2 | 2 | 2 | 2 | 2 | 2 | 20 |
| 14A | 3 | 3 | 3 | 3 | 3 | 3 | 3 | 3 | 3 | 3 | 30 |
| 14B | 1 | 1 | 1 | 1 | 1 | 1 | 1 | 1 | 1 | 1 | 10 |
| 15 | 1 | 1 | 1 | 1 | 1 | 1 | 1 | 1 | 1 | 1 | 10 |
| 15A | 2 | 2 | 2 | 2 | 2 | 2 | 2 | 2 | 2 | 2 | 20 |
| 15B | 3 | 3 | 3 | 3 | 3 | 3 | 3 | 3 | 3 | 3 | 30 |
| 16 | 1 | 1 | 1 | 1 | 1 | 1 | 1 | 1 | 1 | 1 | 10 |
| 16A | 2 | 2 | 2 | 2 | 2 | 2 | 2 | 2 | 2 | 2 | 20 |
| 16B | 3 | 3 | 3 | 3 | 3 | 3 | 3 | 3 | 3 | 3 | 30 |
| 17 | 2 | 2 | 2 | 2 | 2 | 2 | 2 | 2 | 2 | 2 | 20 |
| 17A | 3 | 3 | 3 | 3 | 3 | 3 | 3 | 3 | 3 | 3 | 30 |
| 17B | 1 | 1 | 1 | 1 | 1 | 1 | 1 | 1 | 1 | 1 | 10 |

Les résultats ci-dessus montrent clairement que des effets de synergie entre les polymères (a) et le polymère (b) apparaissent dans les domaines de rapports pondéraux (a)/(b) suivants :

- [0,5 - 3] dans le cadre d'un polymère (a) du type MIRAPOL 175® (voir lotions 1 à 3B) ou du type MIRAPOL 9® (voir lotions 11 à 13B)
- [0,1 - 1,5] dans le cadre d'un polymère (a) du type MIRAPOL A15® (voir lotions 4 à 6B)
- [1,25 - 1,75] dans le cadre d'un polymère (a) du type MIRAPOL AD1® (voir lotions 7 à 10B) ou du type MIRAPOL AZ1 (voir lotions 14 à 17B).

**EXEMPLE 2**

On donne ici un exemple illustrant un shampooing conforme à l'invention.

| | |
|---|---|
| - Lauryl éther sulfate de triéthanolamine (C12/C14 à 70/30) en solution aqueuse à 40% (Empicol TL 40/FL® de chez ALBRIGHT & WILSON) | 15 g MA |
| - Poly (n=6) Dichlorure de N - (diméthylammonio) propyl 1-N'-[3-(éthylèneoxyéthylène diméthylammonio) propylurée] (Mirapol A15® de chez RHÔNE-POULENC) | 1 g MA |
| - Copolymère de chlorure de diméthyldiallylammonium et d'acide acrylique (80/20) en solution aqueuse à 35% (Merquat 280® de chez MERCK) | 1 g MA |
| - Conservateurs, parfums et colorants | qs |
| - HCl qs | pH 5 |
| - Eau qsp | 100 g |

**EXEMPLE 3**

On donne ici un autre exemple de shampooing conforme à l'invention.

| | |
|---|---|
| - Alkyl (C9/C10/C11 à 20/40/40 % en poids) polyglucoside(1,4) en solution aqueuse à 50% (APG 300® de chez HENKEL) | 15 g MA |
| - Chlorure de polyammonium quaternaire (PM : 20 000) en solution aqueuse à 62% (Mirapol 175® de chez RHÔNE-POULENC) | 0,5 g MA |
| - Copolymère de chlorure de diméthyldiallylammonium et d'acide acrylique (80/20) en solution aqueuse à 35% (Merquat 280® de chez MERCK) | 1 g MA |
| - Conservateurs, parfums et colorants | qs |
| - HCl qs | pH 6 |
| - Eau qsp | 100 g |

**EXEMPLE 4**

On donne ici un exemple illustrant un après-shampooing conforme à l'invention.

| | |
|---|---|
| - Chlorure de cétyl triméthylammonium en solution aqueuse à 25% (Dehyquat A® de chez HENKEL) | 3 g MA |
| - Chlorure de polyammonium quaternaire (PM : 50 000) en solution aqueuse à 50% (Mirapol AZ1® de chez RHÔNE-POULENC) | 2 g MA |
| - Copolymère de chlorure de diméthyldiallylammonium et d'acide acrylique (80/20) en solution aqueuse à 35% (Merquat 280® de chez MERCK) | 1,33 g MA |
| - Hydroxyéthylcellulose (Natrosol 250® HHR de chez AQUALON) | 2,5 g |
| - Mélange d'alcools cétylstéarylique et cétylstéarylique oxyéthyléné à 33 moles d'OE (80/20) (Deshconet 390® de chez TENSIA) | 2 g |
| - NaOH qs | pH 7 |
| - Eau qsp | 100 g |

**EXEMPLE 5**

On donne ici un autre exemple d'après-shampooing conforme à l'invention.

| | |
|---|---|
| - Chlorure de béhényltriméthylammonium à 80% dans un mélange eau/isopropanol (Genamin KDM-F® de chez HOECHST) | 2,5 g MA |
| - Chlorure de polyammonium quaternaire (PM : 50 000) en solution aqueuse à 60% (Mirapol AD1® de chez RHÔNE-POULENC) | 1,5 g MA |
| - Copolymère de chlorure de diméthyldiallylammonium et d'acide acrylique (80/20) en solution aqueuse à 35% (Merquat 280® de chez MERCK) | 1 g MA |
| - Mélange d'alcools cétylstéarylique et cétylstéarylique oxyéthyléné à 33 moles d'OE (80/20) (Deshconet 390® de chez TENSIA) | 3 g |
| - HCl qs | pH 4,5 |
| - Eau qsp | 100 g |

**EXEMPLE 6**

On donne ici un exemple illustrant un gel douche conforme à l'invention.

| | |
|---|---|
| - Acide lauryl (C12/C14 à 70/30) éther carboxylique contenant 4,5 moles d'oxyde d'éthylène, en solution aqueuse à 90% (Akypo RLM 45® de chez LAMBERT RIVIERE) | 15 g MA |
| - Lauryl éther sulfate de sodium (C12/C14 à 70/30) contenant 2 moles d'oxyde d'éthylène, en solution aqueuse à 28% (Empicol ESB/3FL® de chez ALBRIGHT & WILSON) | 10 g MA |
| - Chlorure de polyammonium quaternaire (PM : 20 000) en solution aqueuse à 62% (Mirapol 9® de chez RHÔNE-POULENC) | 0,8 g MA |
| - Copolymère de chlorure de diméthyldiallylammonium et d'acide acrylique (80/20) en solution aqueuse à 35% (Merquat 280® de chez MERCK) | 1,2 g MA |
| - Glycérine pure | 1,5 g |
| - Conservateurs, parfums qs | |
| - NaOH qs | pH 7,5 |
| - Eau qsp | 100 g |

**Revendications**

1. Compositions cosmétiques, caractérisées par le fait qu'elles comprennent :

   - au moins un polymère (a) de polyammonium quaternaire constitué d'une succession de motifs répondant à la formule (I) suivante :

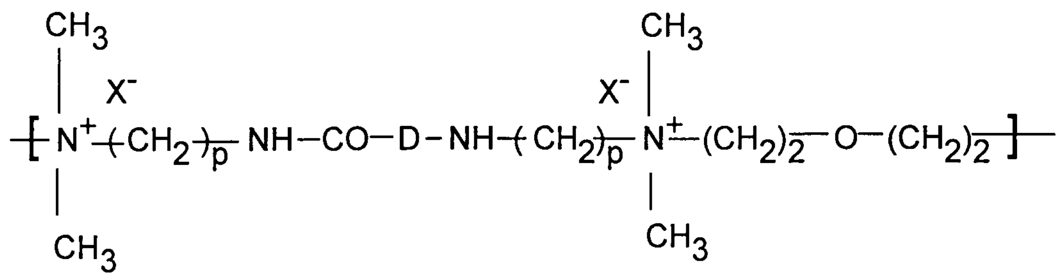

(I)

dans laquelle :

- p désigne un nombre entier variant de 1 à 6 environ,
- D peut être nul ou peut représenter un groupement $-(CH_2)_r-CO-$ dans lequel r désigne un nombre égal à 4 ou à 7,
- $X^-$ est un anion dérivé d'un acide minéral ou organique,

le poids moléculaire dudit polymère (a) étant inférieure à 100 000, de préférence inférieure ou égale à 50 000, et

- au moins un polymère (b) constitué de 70% à 90% en poids environ d'unités diallyldialkylammonium dans lesquelles le radical alkyle contient de 1 à 18 atomes de carbone, et de 30% à 10% en poids environ d'unités acrylique ou méthacrylique,

lesdits polymères (a) et (b) étant présents dans une proportion produisant une activité synergique.

**2.** Compositions selon la revendication 1, caractérisées par le fait que le polymère (a) est choisis parmi les polymères dans lesquels D représente la valeur zéro et X représente un atome de chlore.

**3.** Compositions selon la revendication 1, caractérisées par le fait que le polymère (a) est choisi parmi les polymères dans lesquels D représente un groupement $-(CH_2)_4-CO-$ et X représente un atome de chlore.

**4.** Compositions selon la revendication 1, caractérisées par le fait que le polymère (a) est choisi parmi les polymères dans lesquels D représente un groupement $-(CH_2)_7-CO-$ et X représente un atome de chlore.

**5.** Compositions selon la revendication 1, caractérisées par le fait que le polymère (a) est choisi parmi les copolymères blocs formés de motifs correspondant à ceux indiqués dans les revendications 2 et 3.

**6.** Compositions selon la revendication 1, caractérisées par le fait que le rapport pondéral [polymère (a)/polymère (b)] est compris entre 0,1 et 3.

**7.** Compositions selon la revendication 2, caractérisées par le fait que le rapport pondéral [polymère (a)/polymère (b)] est compris entre 0,1 et 1,5.

**8.** Compositions selon la revendication 3 ou 4, caractérisées par le fait que le rapport pondéral [polymère (a)/polymère (b)] est compris entre 1,25 et 1,75.

**9.** Compositions selon la revendication 5, caractérisées par le fait que le rapport pondéral [polymère (a)/polymère (b)] est compris entre 0,5 et 3.

**10.** Compositions selon l'une quelconque des revendications précédentes, caractérisées par le fait que le polymère (b) présente un poids moléculaire compris entre 50 000 et 10 000 000, de préférence compris entre 200 000 et 5 000 000.

**11.** Compositions selon la revendication 10, caractérisées par le fait que le polymère (b) est un copolymère de chlorure de diallyldiméthylammonium ou de diallyldiéthylammonium et d'acide acrylique, présentant un poids moléculaire compris entre 200 000 et 5 000 000.

**12.** Compositions selon l'une quelconque des revendications précédentes, caractérisées par le fait que le polymère (a) est présent à raison de 0,05% à 4% en poids, de préférence à raison de 0,1% à 3% en poids, par rapport au poids total de la composition.

**13.** Compositions selon l'une quelconque des revendications précédentes, caractérisées par le fait que le polymère (b) est présent à raison de 0,1% à 8% en poids, de préférence à raison de 0,2% à 6% en poids, par rapport au poids total de la composition.

**14.** Compositions selon l'une quelconque des revendications précédentes, caractérisées par le fait qu'elles présentent un pH compris entre 4 et 8, de préférence entre 5 et 7.

**15.** Compositions selon l'une quelconque des revendications précédentes, caractérisées par le fait qu'elles contiennent en outre au moins un agent tensioactif choisi parmi les tensioactifs anioniques, cationiques, non ioniques, amphotères, zwitterioniques et leurs mélanges.

**16.** Compositions selon la revendication 15, caractérisées par le fait que le ou les agents tensioactifs sont présents à une concentration comprise entre 0,1% et 40% en poids, de préférence entre 3% et 40% en poids, et encore plus préférentiellement entre 5% et 30% en poids, par rapport au poids total de la composition.

**17.** Compositions selon l'une quelconque des revendications précédentes, caractérisées par le fait qu'elles contiennent en outre au moins un additif choisi parmi les parfums, les solvants, les agents conservateurs, les agents séquestrants, les agents épaississants, les agents adoucissants, les agents modificateurs de mousse, les agents acidifiants ou alcalinisants, les colorants, les agents modificateurs de viscosité, les agents nacrants, les agents hydratants, les agents anti-pelliculaires, les agents anti-séborrhéiques, les filtres solaires, les (nano)pigments, les silicones volatiles ou non, organomodifiées ou non, les agents de conditionnements différents des polymères a) et b) tels que des composés cationiques polymériques ou non polymériques ou les huiles hydrocarbonées, les protéines.

**18.** Compositions selon l'une quelconque des revendications précédentes, caractérisées par le fait qu'elles se présentent sous forme de shampooings, d'après-shampooings à rincer, de compositions pour la permanente, le défrisage, la coloration ou la décoloration des cheveux, de compositions à rincer à appliquer entre les deux étapes d'une permanente ou d'un défrisage, de compositions lavantes pour le corps, de lotions.

**19.** Utilisation cosmétique d'une composition telle que définie dans l'une quelconque des revendications précédentes pour le lavage ou pour le soin des cheveux et/ou de la peau.

**Claims**

**1.** Cosmetic compositions, characterized in that they comprise:

- at least one quaternary polyammonium polymer (a) consisting of a sequence of units corresponding to the following formula (I):

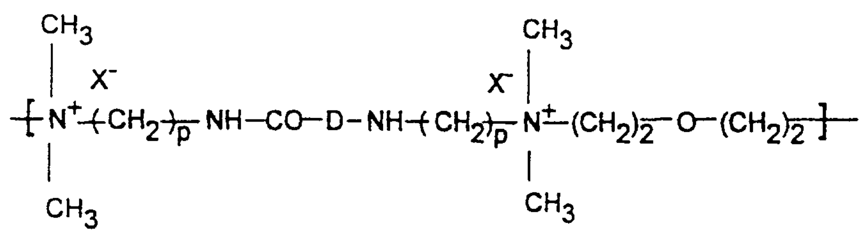

(I)

in which:

- p denotes an integer varying from approximately 1 to 6,
- D may not exist or can represent a $-(CH_2)_r$-CO- group in which r denotes a number equal to 4 or to 7,
- $X^-$ is an anion derived from an inorganic or organic acid,

the molecular mass of the said polymer (a) being less than 100,000 and preferably less than or equal to 50,000, and

- at least one polymer (b) consisting of approximately 70% to 90% by weight of diallyldialkylammonium units in which the alkyl radical contains from 1 to 18 carbon atoms and of approximately 30% to 10% by weight of acrylic or methacrylic units,

the said polymers (a) and (b) being present in a proportion which produces a synergic activity.

**2.** Compositions according to Claim 1, characterized in that the polymer (a) is chosen from the polymers in which D represents the value zero and X represents a chlorine atom.

**3.** Compositions according to Claim 1, characterized in that the polymer (a) is chosen from the polymers in which D represents a $-(CH_2)_4$-CO- group and X represents a chlorine atom.

**4.** Compositions according to Claim 1, characterized in that the polymer (a) is chosen from the polymers in which D represents a $-(CH_2)_7$-CO- group and X represents a chlorine atom.

**5.** Compositions according to Claim 1, characterized in that the polymer (a) is chosen from the block copolymers formed from units corresponding to those indicated in Claims 2 and 3.

**6.** Compositions according to Claim 1, characterized in that the [polymer (a)/polymer (b)] weight ratio is between 0.1 and 3.

**7.** Compositions according to Claim 2, characterized in that the [polymer (a)/polymer (b)] weight ratio is between 0.1 and 1.5.

**8.** Compositions according to Claim 3 or 4, characterized in that the [polymer (a)/polymer (b)] weight ratio is between 1.25 and 1.75.

**9.** Compositions according to Claim 5, characterized in that the [polymer (a)/polymer (b)] weight ratio is between 0.5 and 3.

**10.** Compositions according to any one of the preceding claims, characterized in that the polymer (b) has a molecular weight of between 50,000 and 10,000,000 and preferably between 200,000 and 5,000,000.

**11.** Compositions according to Claim 10, characterized in that the polymer (b) is a copolymer of diallyldimethylammonium or diallyldiethylammonium chloride and of acrylic acid, having a molecular weight of between 200,000 and 5,000,000.

**12.** Compositions according to any one of the preceding claims, characterized in that the polymer (a) is present in a proportion of 0.05% to 4% by weight and preferably in a proportion of 0.1% to 3% by weight with respect to the total weight of the composition.

**13.** Compositions according to any one of the preceding claims, characterized in that the polymer (b) is present in a proportion of 0.1% to 8% by weight and preferably in a proportion of 0.2% to 6% by weight with respect to the total weight of the composition.

**14.** Compositions according to any one of the preceding claims, characterized in that they have a pH of between 4 and 8 and preferably between 5 and 7.

**15.** Compositions according to any one of the preceding claims, characterized in that they additionally contain at least one surface-active agent chosen from anionic, cationic, non-ionic, amphoteric or zwitterionic surface-active agents

and their mixtures.

16. Compositions according to Claim 15, characterized in that the surface-active agent(s) is/are present at a concentration of between 0.1% and 40% by weight, preferably between 3% and 40% by weight and more preferentially still between 5% and 30% by weight with respect to the total weight of the composition.

17. Compositions according to any one of the preceding claims, characterized in that they additionally contain at least one additive chosen from fragrances, solvents, preserving agents, sequestering agents, thickening agents, emollients, foam-modifying agents, acidifying or basifying agents, dyes, viscosity-modifying agents, pearlescence agents, moisturizing agents, anti-dandruff agents, anti-seborrhoeic agents, sunscreens, (nano)pigments, optionally organomodified volatile or nonvolatile silicones, or conditioning agents other than the polymers (a) and (b), such as polymeric or non-polymeric cationic compounds or hydrocarbon oils or proteins.

18. Compositions according to any one of the preceding claims, characterized in that they are provided in the form of shampoos, of rinsing conditioners, of compositions for permanent-waving, straightening, dyeing or bleaching the hair, of rinsing compositions to be applied between the two stages of a permanent wave or of a hair straightening, of washing compositions for the body, or of lotions.

19. Cosmetic use of a composition as defined in any one of the preceding claims for washing or for caring for the hair and/or for the skin.

**Patentansprüche**

1. Kosmetische Zusammensetzungen,
dadurch gekennzeichnet, daß sie enthalten:

- mindestens ein Polymer (a), das eine polymere quaternäre Ammoniumverbindung darstellt, aus aufeinanderfolgenden Einheiten der nachstehenden Formel I

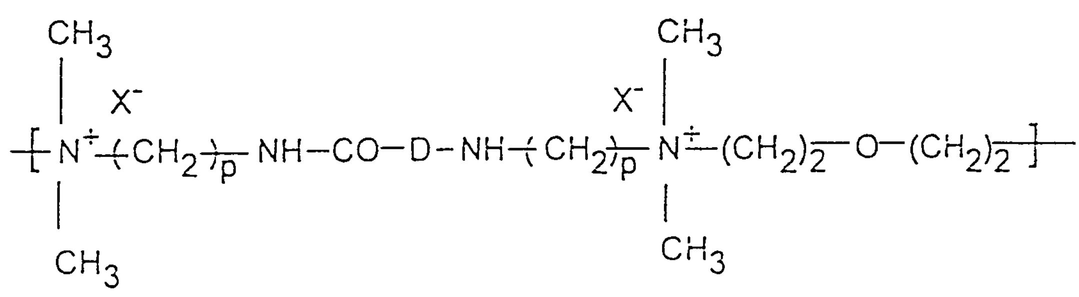

(I)

besteht, in der bedeuten:

- p eine ganze Zahl von etwa 1 bis 6,
- D eine Einfachbindung oder eine Gruppe $-(CH_2)_r-CO-$, in der r 4 oder 7 darstellt, und
- $X^-$ ein von einer anorganischen oder organischen Säure abgeleitetes Anion,

und
eine Molekülmasse von weniger als 100.000 und vorzugsweise höchstens 50.000 aufweist,
sowie

- mindestens ein Polymer (b), das zu etwa 70 bis 90 Gew.-% aus Diallyldialkylammonium-Einheiten, bei denen die Alkylgruppen 1 bis 18 Kohlenstoffatome aufweisen, und zu etwa 30 bis 10 Gew.-% aus Acryl- oder Methacryleinheiten besteht,

wobei die Polymeren (a) und (b) in einem Mengenverhältnis vorliegen, bei dem eine synergistische Wirksamkeit auftritt.

2. Zusammensetzungen nach Anspruch 1, dadurch gekennzeichnet, daß das Polymer (a) unter Polymeren ausgewählt ist, in deren Formel D eine Einfachbindung und X ein Chloratom bedeuten.

3. Zusammensetzungen nach Anspruch 1, dadurch gekennzeichnet, daß das Polymer (a) unter Polymeren ausgewählt ist, in deren Formel D eine Gruppe $-(CH_2)_4-CO-$ und X ein Chloratom bedeuten.

4. Zusammensetzungen nach Anspruch 1, dadurch gekennzeichnet, daß das Polymer (a) unter Polymeren ausgewählt ist, in deren Formel D eine Gruppe $-(CH_2)_7-CO-$ und X ein Chloratom bedeuten.

5. Zusammensetzungen nach Anspruch 1, dadurch gekennzeichnet, daß das Polymer (a) unter Blockcopolymeren ausgewählt ist, die aus Einheiten aufgebaut sind, die den in den Ansprüchen 2 und 3 definierten Einheiten entsprechen.

6. Zusammensetzungen nach Anspruch 1, dadurch gekennzeichnet, daß das Gewichtsverhältnis Polymer (a)/Polymer (b) 0,1 bis 3 beträgt.

7. Zusammensetzungen nach Anspruch 2, dadurch gekennzeichnet, daß das Gewichtsverhältnis Polymer (a)/Polymer (b) 0,1 bis 1,5 beträgt.

8. Zusammensetzungen nach Anspruch 3 oder 4, dadurch gekennzeichnet, daß das Gewichtsverhältnis Polymer (a)/Polymer (b) 1,25 bis 1,75 beträgt.

9. Zusammensetzungen nach Anspruch 5, dadurch gekennzeichnet, daß das Gewichtsverhältnis Polymer (a)/Polymer (b) 0,5 bis 3 beträgt.

10. Zusammensetzungen nach einem der vorhergehenden Ansprüche, dadurch gekennzeichnet, daß das Polymer (b) ein Molekulargewicht von 50.000 bis 10 Millionen und vorzugsweise von 200.000 bis 5 Millionen aufweist.

11. Zusammensetzungen nach Anspruch 10, dadurch gekennzeichnet, daß das Polymer (b) ein Copolymer von Diallyldimethylammoniumchlorid oder Diallyldiethylammoniumchlorid mit Acrylsäure ist und ein Molekulargewicht von 200.000 bis 5 Millionen aufweist.

12. Zusammensetzungen nach einem der vorhergehenden Ansprüche, dadurch gekennzeichnet, daß das Polymer (a) in einem Mengenanteil von 0,05 bis 4 Gew.-% und vorzugsweise in einem Mengenanteil von 0,1 bis 3 Gew.-%, bezogen auf das Gesamtgewicht der Zusammensetzung, vorliegt.

13. Zusammensetzungen nach einem der vorhergehenden Ansprüche, dadurch gekennzeichnet, daß das Polymer (b) in einem Mengenanteil von 0,1 bis 8 Gew.-% und vorzugsweise in einem Mengenanteil von 0,2 bis 6 Gew.-%, bezogen auf das Gesamtgewicht der Zusammensetzung, vorliegt.

14. Zusammensetzungen nach einem der vorhergehenden Ansprüche, dadurch gekennzeichnet, daß sie einen pH-Wert im Bereich von 4 bis 8 und vorzugsweise im Bereich von 5 bis 7 aufweisen.

15. Zusammensetzungen nach einem der vorhergehenden Ansprüche, dadurch gekennzeichnet, daß sie ferner mindestens ein grenzflächenaktives Mittel enthalten, das unter anionischen, kationischen, nichtionischen, amphoteren und zwitterionischen grenzflächenaktiven Mitteln sowie deren Gemischen ausgewählt ist.

16. Zusammensetzungen nach Anspruch 15, dadurch gekennzeichnet, daß das oder die grenzflächenaktiven Mittel in einer Konzentration von 0,1 bis 40 Gew.-%, vorzugsweise in einer Konzentration von 3 bis 40 Gew.-% und noch bevorzugter in einer Konzentration von 5 bis 30 Gew.-%, bezogen auf das Gesamtgewicht der Zusammensetzung, vorliegen.

17. Zusammensetzungen nach einem der vorhergehenden Ansprüche, dadurch gekennzeichnet, daß sie ferner mindestens ein Additiv enthalten, das ausgewählt ist unter Parfums, Lösungsmitteln, Konservierungsmitteln, Sequestrierungsmitteln, Verdickungsmitteln, Mitteln für die Geschmeidigkeit, Schaumregulatoren, Ansäuerungsmitteln oder Alkalinisierungsmitteln, Färbemitteln, Viskositätsregulatoren, Perlglanzmitteln, Feuchthaltemitteln, Antischuppenmitteln, Antiseborrhoemitteln, Sonnenschutzfiltern, (Nano)Pigmenten, flüchtigen oder nichtflüchtigen, organomodifizierten oder nicht organomodifizierten Siliconen, von den Polymeren (a) und (b) verschiedenen Konditionierungsmitteln, wie polymeren oder nichtpolymeren kationischen Verbindungen, sowie Kohlenwasserstoffölen und Proteinen.

18. Zusammensetzungen nach einem der vorhergehenden Ansprüche, dadurch gekennzeichnet, daß sie in Form von Shampoos, Haarspülungen zur Anwendung nach der Haarwäsche, Zusammensetzungen für Dauerwellen, Zusammensetzungen zur Entkräuselung von Haaren, Haarfärbemitteln oder Haarentfärbemitteln, Zusammensetzungen zur Haarspülung zur Anwendung zwischen den beiden Stufen einer Dauerwellenbehandlung oder einer Haarentkräuselungsbehandlung, Zusammensetzungen zum Waschen des Körpers oder Lotionen vorliegen.

19. Kosmetische Verwendung einer Zusammensetzung wie in einem der vorhergehenden Ansprüche definiert zum Waschen oder zur Pflege der Haare und/oder der Haut.